# EUROPEAN PATENT APPLICATION

(11) **EP 3 646 898 A1**
(43) Date of publication of application: **06.05.2020**
(21) Application number: 19201310.0
(22) Date of filing: 13.11.2015
(51) Int. Cl.: A61L 27/18, A61L 27/54, A61L 27/58, A61L 31/08, A61L 31/10, A61L 31/14, A61L 31/16, A61K 9/00, A61L 27/50

(54) **DELIVERY OF IGF-1 IN MYOCARDIAL INFARCTION**

(30) Priority: 14.11.2014 US 201462080231 P
(62) Divisional of application: 15805125.0
(71) Applicant: University College Cork-National University of Ireland, Cork, Cork (IE)
(72) Inventor: Caplice, Noel, Mallow, Co. Cork P51 YP2C (IE)
(74) Representative: Purdylucey Intellectual Property

(57) **Abstract**

Described herein are methods that result in less cell death resulting from myocardial infarction than would otherwise occur in an individual who has suffered myocardial infarction. Also described are compositions useful in reducing cell death post myocardial infarction.

## Description

### CROSS REFERENCE TO RELATED APPLICATIONS

This application claims the benefit of the filing date of U.S. Provisional Application No. 62/080,231, filed November 14, 2014, which is incorporated herein in its entirety by reference.

### BACKGROUND OF INVENTION

Cardiomyocyte death post myocardial infarction (MI) is associated with significant morbidity and mortality due to shock, heart failure and lethal arrhythmia. Additional approaches to reducing the effects of MI are needed.

### SUMMARY OF INVENTION

Cytoprotection of cells in the infarct and border zones early post MI offers a potentially propitious target, but to date in vivo cardiomyocyte anti-apoptosis therapy has been limited by available delivery strategies, such as delivery of recombinant protein derived agents with short half-life in the circulation or problematic as a result of systemic release and inactivation. The timeframe for acute cardiomyocyte death post MI can extend out to 2-3 days post infarction and, therefore, more sustained therapeutic cytoprotection over this timeframe is desirable.

Described herein are methods of cytoprotection of cells (cardiomyocytes) post myocardial infarction. The methods result in less cell death resulting from myocardial infarction than would otherwise occur in an individual who has suffered myocardial infarction. Also described are compositions useful in reducing cell death post myocardial infarction.

### Injection of composition comprising IGF-1

One embodiment described herein is a method of reducing cell death, also referred to as increasing cytoprotection of cells (cardiomyocytes), in heart muscle/tissue resulting from myocardial infarction in an individual in whom myocardial infarction (MI) has occurred. This embodiment comprises administering Insulin-Like Growth Factor-1 (IGF-1) by intracoronary injection (also referred to as intravascular injection or injection into coronary artery) in a location that results in delivery of sufficient IGF-1 to the area of risk (e.g., heart muscle/tissue at or near the site of MI, such as in the infarct and border zones) in an individual in whom myocardial infarct has occurred. Also described are slow-release compositions useful in reducing cell death in heart muscle/tissue resulting from myocardial infarction, which comprise IGF-1 and a (at least one, one or more) biocompatible slow-release component.

One embodiment of the method of administering a composition comprising IGF-1 is a method of injecting IGF-1, particularly by intracoronary injection, in a composition from which IGF-1 is released in sufficient amount, typically within 72 hours after injection, to reduce the extent to which myocardial cell death occurs. In one embodiment of the method of administering a composition comprising IGF-1, IGF-1 is administered in a slow release composition that comprises IGF-1. In this embodiment, IGF-1 is administered by injection (typically, a single intracoronary injection), to an individual in whom myocardial infarction has occurred, of a slow release composition that comprises (a) a therapeutically effective amount of IGF-1 and (b) a biocompatible slow release component from which IGF-1 is released. Injection occurs within 72 hours after occurrence of myocardial infarction and in specific embodiments, a single injection of the slow release composition is administered within 72 hours, within 48 hours, within 24 hours or less (e.g., within 5 minutes, within 10 minutes, within 30 minutes, within 1 hour, within 2 hours, within 5 hours, within 8 hours, within 12 hours, within 18 hours) after myocardial infarction. A therapeutically effective amount of IGF-1 is an amount that reduces the extent to which cell death occurs in heart muscle/tissue (cardiomyocyte death); cell death occurs to a lesser extent than would occur in the absence of injected IGF-1. Intracoronary delivery, which results in intracoronary infusion into the coronary circulation, is used to deliver a therapeutically effective amount of IGF-1 to damaged cardiac muscle/tissue and reduce the extent to which damage (e.g., cell death) occurs; less cell death occurs than would occur in the absence of IGF-1 administered by intracoronary delivery. Intracoronary injection is carried out using known approaches, such as by coronary circulation catheter.

The amount of IGF-1 administered is from about 1.0ng of IGF-1 to about 100ng and in specific embodiments, the amount is from about 1.0ng-2.0ng, from about 1.0ng-5.0ng, from about 1.0ng-10ng, from about 1.0ng-20ng, from about 1.0ng-30ng, from about 1.0ng-40ng, from about 1.0ng-60ng, from about 1.0ng-80ng. In other specific embodiments, 1.0ng, 1.5ng, 2.0ng, 3.0ng, 4.0ng, 5.0ng, 6.0ng, 7.0ng, 8.0ng, 9.0ng, 10.0ng, 1 1.0ng, 12.0ng, 13.0ng, 14.0ng or 15.0ng IGF-1 is administered. In each embodiment, a slow release composition comprising IGF-1 in an amount sufficient to result in release of the desired amount (1.0ng of IGF-1 to about 100ng) is injected, typically as a single intracoronary injection. For example, the slow release composition releases from about 1.0ng of IGF-1 to about 100ng of IGF-1 into cardiac muscle after intracoronary injection. A slow release composition comprises at least one slow release component and, in some embodiments, includes a single (one type of) slow release component, such as a slow release polymer. In some embodiments, IGF-1 is embedded or conjugated in polymer matrices in order to control release of IGF-1. The use of polymers as drug delivery systems has been described in the art. See e.g., Liechty W.B., et al., "Polymers for Drug Delivery Systems." Annu Rev Chem biomol Eng. 2010; 1:149-173; Vilar G., et al., "Polymers and Drug Delivery Systems." Current Drug Delivery,2012 Jul; 9(4):367-94; Makadia H. K., et al., "Poly Lactic-co-Glycolic Acid (PLGA) as Biodegradable Controlled Drug Delivery Carrier." Polymers (Basel), Sep 1, 2011; 3(3): 1377-1397. Each of the cited references is incorporated herein by reference.

In some embodiments, the slow release polymer is a drug-polymer conjugate. IGF-1 can be covalently bound to the polymer backbone. These types of drug-polymer conjugates can improve cell specificity to achieve optimal release of UGF-1 at the proposed target. In other embodiments, IGF-1 can be embedded or encapsulated in the slow release polymer to control the distribution at the correct site over time. In some embodiments, the slow release polymers form supramolecular structures that are suitable for retaining IGF-1 and allow controlled release. In some embodiments the interactions between the polymer chains are noncovalent (e.g., electrostatic and hydrogen bonds) and in other embodiments the interactions between the polymer chains are covalent (e.g., cross-linked polymers). The integrity and behavior of the slow release polymer structures in physiological conditions can allow the controlled release of IGF-1. In certain embodiments, release of IGF-1 from the slow release polymer is controlled by swelling. Hydration of the polymer causes an increase in the volume of the polymeric structure and the resulting increase in pore size allows diffusion of the aqueous medium within the polymeric structure and thus the release of the drug. In other embodiments, IGF-1 release can be achieved through polymer degradation. Gradual hydration of the polymeric structure can cause hydrolytic degradation with release of its contents. This degradation depends on the stability of the polymer chain linkages in body fluids. Alternatively, IGF-1 release can be achieved by diffusion. The IGF-1 may slowly diffuse through the voids of the slow release polymer. These polymers can be fabricated as matrices in which the IGF-1 is uniformly distributed.

There are many ways in which IGF-1 may be embedded or encapsulated in a slow release polymer. In some embodiments, IGF-1 is embedded in/placed on the surface of a micro-particle or a nano-particle. Typically, micro-particles and nano-particles release IGF-1 by diffusion through the pores of the polymer. In other embodiments, IGF-1 is encapsulated in a micro-capsule or a nano-capsule. Degradation of the polymeric micro-capsule or nano-capsule is generally required for release of the encapsulated IGF-1. In other embodiments, IGF-1 is embedded or encapsulated in a capsosome or a micelle. Capsosomes consist of a drug carrier with an external core and liposomes inside, thus allowing various compartments inside its structure micelles. Micelles are composed of amphipathic linear polymers that are spontaneously formed by self-assembly in water; IGF-1 is encapsulated in the hydrophobic core.

In specific embodiments, slow release biodegradable polymers are used. In some embodiments, the slow release polymers are synthetic biodegradable polymers, which may include relatively hydrophobic materials, such as α-hydroxy acids (a family that includes poly lactic-co-glycolic acid, PLGA), polyanhydrides. In other embodiments, the slow release polymers are naturally occurring polymers, such as complex sugars (hyaluronan, chitosan) and inorganics (hydroxyapatite). Polyester PLGA is a copolymer of poly lactic acid (PLA) and poly glycolic acid (PGA). PLGA is a well-defined biomaterial for drug delivery with respect to design and performance. Poly lactic acid contains an asymmetric α-carbon which is typically described as the D or L form in classical stereochemical terms and sometimes as R and S form, respectively. The enantiomeric forms of the polymer PLA are poly D-lactic acid (PDLA) and poly L-lactic acid (PLLA). PLGA is generally an acronym for poly D,L-lactic-co-glycolic acid where D- and L- lactic acid forms are in equal ratio.

In certain embodiments, copolymers of PLGA are used. A wide variety of block copolymers of polyesters with poly ethylene glycol (PEG) are known in the art. In certain embodiments, PLGA/PEG block copolymers are processed as diblock (PLGA-PEG) or triblock molecules with both ABA (PLGA-PEG-PLGA) and BAB (PEG-PLGA-PEG) types. In certain embodiments, diblock polymers are used. In diblock types, PEG chains orient themselves towards the external aqueous phase in micelles, thus surrounding the encapsulated species. This layer of PEG acts as a barrier and reduces the interactions with foreign molecules by steric and hydrated repulsion, giving enhanced shelf stability. In certain embodiments triblock polymers are used. Triblock copolymers of both ABA and BAB type can act as a thermogel with an A-block covalently coupled with a B-block via ester link. The copolymer is typically a free flowing solution at low temperature and can form a high viscosity gel at body temperature. These temperature-responsive copolymers, PLGA-PEG-PLGA or PEG-PLGA-PEG, are a kind of block copolymer composed of hydrophobic PLGA segments and hydrophilic PEG segments. The hydrophobic PLGA segments form associative crosslinks and the hydrophilic PEG segments allow the copolymer molecules to stay in solution. At lower temperatures, hydrogen bonding between hydrophilic PEG segments and water molecules dominates the aqueous solution, resulting in their dissolution in water. As the temperature increases, the hydrogen bonding becomes weaker, while hydrophobic forces among the PLGA segments are strengthened, leading to solution-gel transition. In some embodiments, release of IGF-1 from both ABA and BAB copolymers can occur by drug diffusion from the hydrogel during the initial release phase. In other embodiments, IGF-1 is released by erosion of the hydrogel matrix during the later phase.

In a specific embodiment the slow release component is a biodegradable, semicrystalline, phase separated thermoplastic multi-block copolymer, such as described in United States Patent Application 14/233,961 (publication number US 2014/0199385). As one example, the slow release polymers can be composed of a crystalline L-lactide-based hard segment with a melting point (Tm) and a hydrophilic poly(ethylene glycol) (PEG)-based segment A having a glass transition temperature (Tg) below body temperature under physiological conditions.

In some embodiments, the slow release composition includes two or more, different slow release components, each of which releases IGF-1 at a different rate or time after injection. For example, a first slow release component releases the IGF-1 it contains within about 24 hours after injection and a second, different slow release component releases the IGF-1 it contains over a longer period, such as 48 hours, or starting at a later time after injection, such as starting at about 24 hours after injection.

Slow release components can be any biocompatible material that is suitable for introduction into an individual (e.g., a human or other mammal), such as into a coronary blood vessel (e.g., a coronary artery) or other blood vessel and from which IGF-1 is released over time. Slow release compositions comprising a (at least one, one or more) slow release component and IGF-1 can be introduced into an individual by, for example, intracoronary injection or by insertion of a stent or other surface coated with or otherwise bearing IGF-1 that is released into the blood or tissues of the recipient. The stent or other surface can comprise IGF-1 in a biocompatible agent from which it is released after introduction into an individual, such as after insertion into the circulatory system (e.g., into a coronary artery or other artery).The stent or other surface can be coated with layers of biocompatible materials, such as through the use of atomic layer deposition (ALD). ALD can be used to coat Angstrom thick monolayers of biocompatible molecules useful for drug capture and controlled release of the captured drug (e.g., capture and controlled release of IGF-1).

A wide variety of biocompatible polymers may be used. Such biocompatible materials are known in the art and have been described. See, e.g., Prisell et al., U.S. Patent Number 5,470,829, which is incorporated herein by reference. Slow release components include, but are not limited to Polyglycolide (PGA), Copolymers of glycolide, Glycolide/L-lactide copolymers (PGA/PLLA), Glycolide/trimethylene carbonate copolymers (PGS/TMC), Polylactides (PLA), Stereo-copolymers of PLA, Poly-L-lactide (PLLA), Poly-DL-lactide (PDLLA), L-lactide/DL-lactide copolymers, Copolyers of PLA, Lactide/tetramethylglycolide copolymers, Lactide/trimethylene carbonate copolymers, Lactide/a-valerolactone copolymers, Lactide/ε-caprolactone copolymers, Hyaluronic acid and its derivatives, Polydepsipeptides, PLA/polyethylene oxide copolymers, Unsymmetrical 3,6-substitute poly-1,4-dioxane-2,5-di-ones, Poly-phydroxybutyrate (PHBA), PHBA A/β-hydroxyvalerate copolymers (PHBA/HVA), Poly-p-dioxanone (PDS), Poly-α-valerolactone, Poly-e-caprolactone, Methylmethacrylate-N-vinyl pyrrolidine copolymers, Polyesteramides, Polyesters of oxalic acid, Polydihydropyranes, Polyalkyl-2-cyanoacrylates, Polyurethanes (PU), Polyvinylalcohol (PVA), Polypeptides, Poly-β-malic acid (PMLA), Poly-β-alcanoic acids and alginates.

A further embodiment is a method of reducing cell death in heart muscle/tissue resulting from myocardial infarction, comprising administering a single injection (by intracoronary route) of a slow-release composition that comprises IGF-1 and a (at least one, one or more) slow release component within 72 hours after myocardial infarction, wherein the slow-release composition releases IGF-1 in an amount sufficient to reduce the extent to which cell death occurs in heart muscle/tissue.

In the method, IGF-1 is administered by injection (typically, a single intracoronary injection) at or near the site of myocardial infarction (e.g., in the infarct and border zones) in an individual in whom myocardial infarction has occurred, of a slow release composition that comprises (a) a therapeutically effective amount of IGF-1 and (b) a biocompatible slow release component from which IGF-1 is released. Injection occurs within 72 hours after occurrence of myocardial infarction and in specific embodiments, a single injection of the slow release composition is administered within 72 hours, within 48 hours, 24 hours or less (e.g., within 5 minutes, within 10 minutes, within 30 minutes, within 1 hour, within 2 hours, within 5 hours, within 8 hours, within 12 hours, within 18 hours) after myocardial infarction. A therapeutically effective amount of IGF-1 is an amount that reduces the extent to which cell death occurs in heart muscle/tissue; cell death (cardiomyocyte death) occurs to a lesser extent than would occur in the absence of injected IGF-1.

The amount of IGF-1 administered is from about 1.0ng of IGF-1 to about 100ng and in specific embodiments, the amount is from about 1.0ng-2.0ng, from about 1.0ng-5.0ng, from about 1.0ng-10ng, from about 1.0ng-20ng, from about 1.0ng-30ng, from about 1.0ng-40ng, from about 1.0ng-60ng, from about 1.0ng-80ng. In other specific embodiments, 1.0ng, 1.5ng, 2.0ng, 3.0ng, 4.0ng, 5.0ng, 6.0ng, 7.0ng, 8.0ng, 9.0ng, 10.0ng, 11.0ng, 12.0ng, 13.0ng, 14.0ng or 15.0ng IGF-1 is administered. In each embodiment, a slow release composition comprising IGF-1 in an amount sufficient to result in release of the desired amount (1.0ng of IGF-1 to about 100ng) is injected, typically as a single injection. For example, the slow release composition releases from about 1.0ng of IGF-1 to about 100ng of IGF-1 into cardiac muscle after intracoronary injection.

A slow release composition comprises at least one slow release component and, in some embodiments, includes a single slow release component. Such non-limiting examples include a poly(DL-lactide-co-PEG₁₀₀₀), a poly(DL-lactide-co-PEG₂₀₀₀), a poly(DL-lactide-co-PEG₃₀₀₀), a poly(DL-lactide-co-PEG₄₀₀₀), a poly(DL-lactide-co-PEG₅₀₀₀), a poly(ε-caprolactone-co-PEGiooo), a poly(ε-caprolactone-co-PEG₂₀₀₀), a poly(ε-caprolactone-co-PEG₃₀₀₀), a poly(ε-caprolactone-co-PEG₄₀₀₀), a poly(ε-caprolactone-co-PEG₅₀₀₀), a ε-Caprolactone-co-PEG-co- ε-Caprolactone (CPC), a D,L-lactide-co-PEG-co-D,L-lactide (LPL), or any polymer described in United States Patent Application 14/233,961, published as US 2014/0199385 on July 17, 2014. In other embodiments the slow release polymer is 50CP30L40-LL40 (available from Innocore Pharmaceuticals). In some embodiments, the slow release composition includes two or more, different slow release components, each of which releases IGF-1 at a different rate or at a different time after injection. For example, a first slow release component releases the IGF-1 it contains within about 24 hours after injection and a second, different slow release component releases the IGF-1 it contains over a longer period, such as 48 hours or 72 hours, or starting at a later time after injection, such as starting at about 24 hours after injection.

Slow release components can be any biocompatible material that is suitable for intracoronary injection and from which IGF-1 is released over time. They include, but are not limited to, a poly(DL-lactide-co-PEG₁₀₀₀), a poly(DL-lactide-co-PEG₂₀₀₀), a poly(DL-lactide-co-PEG₃₀₀₀), a poly(DL-lactide-co-PEG₄₀₀₀), a poly(DL-lactide-co-PEG₅₀₀₀), a poly(ε-caprolactone-co-PEGiooo), a poly(ε-caprolactone-co-PEG₂₀₀₀), a poly(ε-caprolactone-co-PEG₃₀₀₀), a poly(ε-caprolactone-co-PEG₄₀₀₀), a poly(ε-caprolactone-co-PEG₅₀₀₀), a ε-Caprolactone-co-PEG-co- ε-Caprolactone (CPC), a D,L-lactide-co-PEG-co-D,L-lactide (LPL), or any polymer described in United States Patent Application 14/233,961, published as US 2014/0199385 on July 17, 2014. In other embodiments the slow release polymer is 50CP30L40-LL40 (available from Innocore Pharmaceuticals).

Another embodiment is a method of delivering IGF-1 to the site of a myocardial infarct comprising injecting into a coronary artery a slow release composition comprising (a) a therapeutically effective amount of IGF-1 and (b) a slow release component in/vicinal to the site of myocardial infarct in an individual (in whom a myocardial infarct has occurred) within 72 hours after occurrence of the myocardial infarct.

In the method, IGF-1 is delivered to the site of a myocardial infarct by intracoronary injection (typically, a single injection, such as at or near the infarct and border zones) in an individual in whom myocardial infarction has occurred, of a slow release composition that comprises (a) a therapeutically effective amount of IGF-1 and (b) a biocompatible slow release component from which IGF-1 is released. Injection occurs within 72 hours after occurrence of the myocardial infarct and in specific embodiments, a single injection of the slow release composition is administered within 72 hours, within 48 hours, 24 hours or less (e.g., within 5 minutes, within 10 minutes, within 30 minutes, within 1 hour, within 2 hours, within 5 hours, within 8 hours, within 12 hours, within 18 hours) after myocardial infarction. A therapeutically effective amount of IGF-1 is an amount that reduces the extent to which cell death occurs in heart muscle/tissue; cell death (cardiomyocyte death) occurs to a lesser extent than would occur in the absence of injected IGF-1.

The amount of IGF-1 administered is from about 1.0ng of IGF-1 to about 100ng and in specific embodiments, the amount is from about 1.0ng-2.0ng, from about 1.0ng-5.0ng, from about 1.0ng-10ng, from about 1.0ng-20ng, from about 1.0ng-30ng, from about 1.0ng-40ng, from about 1.0ng-60ng, from about 1.0ng-80ng. In other specific embodiments, 1.0ng, 1.5ng, 2.0ng, 3.0ng, 4.0ng, 5.0ng, 6.0ng, 7.0ng, 8.0ng, 9.0ng, 10.0ng, 1 1.0ng, 12.0ng, 13.0ng, 14.0ng or 15.0ng IGF-1 is administered. In each embodiment, a slow release composition comprising IGF-1 in an amount sufficient to result in release of the desired amount (1.0ng of IGF-1 to about 100ng) is injected, typically as a single injection. For example, the slow release composition releases from about 1.0ng of IGF-1 to about 100ng of IGF-1 into cardiac muscle after intracoronary injection.

A slow release composition comprises at least one slow release component and, in some embodiments, includes a single (one type of) slow release component, such as a poly(DL-lactide-co-PEG₁₀₀₀), a poly(DL-lactide-co-PEG₂₀₀₀), a poly(DL-lactide-co-PEG₃₀₀₀), a poly(DL-lactide-co-PEG₄₀₀₀), a poly(DL-lactide-co-PEG₅₀₀₀), a poly(ε-caprolactone-co-PEG₁₀₀₀), a poly(ε-caprolactone-co-PEG₂₀₀₀), a poly(ε-caprolactone-co-PEG₃₀₀₀), a poly(ε-caprolactone-co-PEG₄₀₀₀), a poly(ε-caprolactone-co-PEG₅₀₀₀), a ε-Caprolactone-co-PEG-co- ε-Caprolactone (CPC), a D,L-lactide-co-PEG-co-D,L-lactide (LPL), or any polymer described in United States Patent Application 14/233,961 (publication number US 2014/0199385). In other embodiments the slow release polymer is 50CP30L40-LL40 (available from Innocore Pharmaceuticals). In some embodiments, the slow release composition includes two or more, different slow release components, each of which releases IGF-1 at a different rate or time after injection. For example, a first slow release component releases the IGF-1 it contains within about 24 hours after injection and a second, different slow release component releases the IGF-1 it contains over a longer period, such as 48 hours or 72 hours, or starting at a later time after injection, such as starting at about 24 hours after injection.

Slow release components can be any biocompatible material suitable for introduction into intracoronary injection, from which IGF-1 is released over time or introduction into a coronary artery by means of insertion of a stent or other device from which IGF-1 is released over time. They include, but are not limited to, a poly(DL-lactide-co-PEG₁₀₀₀), a poly(DL-lactide-co-PEG₂₀₀₀), a poly(DL-lactide-co-PEG₃₀₀₀), a poly(DL-lactide-co-PEG₄₀₀₀), a poly(DL-lactide-co-PEG₅₀₀₀), a poly(ε-caprolactone-co-PEG₁₀₀₀), a poly(ε-caprolactone-co-PEG₂₀₀₀), a poly(ε-caprolactone-co-PEG₃₀₀₀), a poly(ε-caprolactone-co-PEG₄₀₀₀), a poly(ε-caprolactone-co-PEG₅₀₀₀), a ε-Caprolactone-co-PEG-co- ε-Caprolactone (CPC), a D,L-lactide-co-PEG-co-D,L-lactide (LPL), or any polymer described in United States Patent Application 14/233,961 (publication number US 2014/0199385). In other embodiments the slow release polymer is 50CP30L40-LL40 (available from Innocore Pharmaceuticals.

In a second embodiment of the method of administering a composition comprising IGF-1, at least 1.10ng IGF-1 (e.g., 1.25ng IGF-1 or more) is administered by intracoronary injection; the composition can comprise any biocompatible carrier suitable for intracoronary injection, such as by coronary circulation catheter. In this embodiment, the composition comprises from about 1.10ng IGF-1 to about 1000ng IGF-1 and is administered in a single injection, typically within 72 hours after MI, or in multiple injections, the first of which is typically administered within 72 hours after MI.

### Delivery of modified mRNA

A second embodiment is a method of reducing cell death, also referred to as increasing cytoprotection of cells (cardiomyocytes), in heart muscle/tissue resulting from myocardial infarction, in an individual in whom myocardial infarction (MI) has occurred, which relies on a synthetic chemically modified mRNA (modRNA) to drive delivery of insulin-like growth factor-1 (IGF-1) within the area at risk, such as heart muscle/tissue at or near the site of myocardial infarction (e.g., in the infarct and border zones) in an individual in whom myocardial infarction has occurred. The method is a method of controlled release of IGF-1 into heart muscle/tissue in which a chemically modified RNA-based bioactive nanoparticle is administered to an individual for transient IGF-1 derived cytoprotective therapy, which results in reduced cell death after myocardial infarction.

Synthetic modRNA exhibits auspicious delivery and expression characteristics with respect to targeting of the myocardium, mediating efficient and extended (for days) protein expression in cardiomyocytes without causing an immune response. Transient modRNA expression of a cytoprotective factor, IGF-1, can be induced in cardiomyocytes following hypoxia and also in the setting of experimental acute MI. Delivery of modRNA (e.g., modRNA-IGF-1) into cardiomyocytes is efficient in providing transient expression of intracellular and secreted IGF-1 at picogram to nanogram levels in vitro. In vivo delivery of modRNA resulted in expression of the modRNA in primary mouse heart slices and expression efficiency was enhanced under hypoxia-induced apoptotic conditions. Following mod RNA-IGF-1 treatment, increased levels of intracellular and secreted IGF-1 promoted cardiomyocyte survival, reducing the high level of TUNEL positive cells (cells that undergo subsequent apoptosis.

As shown herein, modRNA-IGF-1 therapy can target an expanded temporal window for cytoprotective intervention in the setting of myocardial infarction. ModRNA-IGF-1 induced down-stream IGF-1-associated signaling. Use of a chemically modified RNA-based bioactive nanoparticle for transient IGF1 derived cytoprotective therapy is a new approach to control the release of IGF-1 and reduce cell death, such as results from MI.

Also the subject of this disclosure is a composition useful in the present method of reducing cell death in an individual (e.g., a human or other mammal) after MI by administering to the individual chemically modified RNA (such as chemically modified RNA-based bioactive nanoparticles) that results in controlled release of IGF-1 into heart muscle/tissue and reduced cell death (less cell death than would occur in the absence of transient expression of IGF-1). The method is a method for transient IGF-1 derived cytoprotective therapy, in an individual in whom an MI has occurred, in which modified RNA (e.g., modified IGF-1 encoding RNA) is administered to an individual for transient IGF-1 derived cytoprotective therapy, which results in reduced cell death after myocardial infarction. In a specific embodiment, modified RNA, such as modified IGF-1 encoding RNA is administered as a chemically modified RNA-based bioactive nanoparticle

### Slow release composition comprising IGF-1

Also described herein is a slow release composition comprising IGF-1 and at least one biocompatible slow release component. The composition releases from about 1.0ng of IGF-1 to about 100ng of IGF-1 after intracoronary injection within 5 minutes, within 10 minutes, within 30 minutes, within 1 hour, within 2 hours, within 5 hours, within 8 hours, within 12 hours, within 18 hours, within 24 hours, within 36 hours, within 48 hours, within 60 hours or within 72 hours after occurrence of myocardial infarct. In a further embodiment, the composition releases from about 1.0ng of IGF-1 to about 100ng of IGF-1, after introduction into an individual on a stent or other surface coated with or otherwise bearing the slow release composition, within 5 minutes, within 10 minutes, within 30 minutes, within 1 hour, within 2 hours, within 5 hours, within 8 hours, within 12 hours, within 18 hours, within 24 hours, within 36 hours, within 48 hours, within 60 hours or within 72 hours after occurrence of myocardial infarct.

The slow release composition comprises IGF-1 in an amount sufficient to result in release of the desired amount (1.0ng of IGF-1 to about 100ng) into cardiac muscle/tissue, as the result of a single intracoronary injection. For example, the slow release composition releases from about 1.0ng of IGF-1 to about 100ng of IGF-1 into cardiac muscle after injection into coronary circulation. In a further embodiment, the slow release composition comprises IGF-1 in an amount sufficient to result in release of a desired amount (e.g., 1.0ng of IGF-1 to about 100ng) into cardiac muscle/tissue, as the result of introduction of a stent or other surface coated with or otherwise bearing the slow release composition. For example, the slow release composition releases from about 1.0ng of IGF-1 to about 100ng of IGF-1 into cardiac muscle after injection into coronary circulation or introduction of a stent or other surface coated with or otherwise bearing the slow release composition.

In specific embodiments, the slow release composition releases from about 1.0ng of IGF-1 to about 100ng of IGF-1 into cardiac muscle within 48 hours after injection into coronary circulation. In further specific embodiments, the slow release composition releases from about 1.0ng of IGF-1 to about 100ng of IGF-1 into cardiac muscle within 48 hours after introduction of a stent or other surface coated with or otherwise bearing the slow release composition.

In specific embodiments, the composition releases from about 1.0ng-2.0ng, from about 1.0ng-5.0ng, from about 1.0ng-10ng, from about 1.0ng-20ng, from about 1.0ng-30ng, from about 1.0ng-40ng, from about 1.0ng-60ng, from about 1.0ng-80ng or from about 1.0ng-100ng of IGF-1 within 48 hours after intracoronary injection. In further embodiments, the composition releases from about 1.0ng- about 20ng or from about 1.0ng - about 15ng of IGF-1 into cardiac muscle within up to 72 hours (up to within any of the following: 5 minutes, 10 minutes, 30 minutes, 1 hour, 2 hours, 5 hours, 8 hours, 12 hours, 18 hours, 24 hours, 36 hours, 48 hours, 60 hours) after injection into coronary circulation, such as by means of a coronary circulation catheter. The slow release composition comprises at least one slow release component and, in some embodiments, includes one (a single type of) slow release component. In further embodiments, the composition comprises at least two, different slow release components, each of which releases IGF-1 at a different rate or time after intracoronary injection, such as by means of a coronary circulation catheter. For example, a first slow release component releases the IGF-1 it contains within about 24 hours after injection and a second, different slow release component releases the IGF-1 it contains over a longer period, such as 48 hours or 72 hours, or starting at a later time after injection, such as starting at about 24 hours after intravascular injection.

In specific embodiments, the composition releases from about 1.0ng-2.0ng, from about 1.0ng-5.0ng, from about 1.0ng-10ng, from about 1.0ng-20ng, from about 1.0ng-30ng, from about 1.0ng-40ng, from about 1.0ng-60ng, from about 1.0ng-80ng or from about 1.0ng-100ng of IGF-1 within 48 hours after introduction into an individual of a stent or other surface coated with or otherwise bearing the slow release composition. In further embodiments, the composition releases from about 1.0ng- about 20ng or from about 1.0ng - about 15ng of IGF-1 into cardiac muscle within up to 72 hours (up to within any of the following: 5 minutes, 10 minutes, 30 minutes, 1 hour, 2 hours, 5 hours, 8 hours, 12 hours, 18 hours, 24 hours, 36 hours, 48 hours, 60 hours) after introduction into an individual of a stent or other surface coated with or otherwise bearing the slow release composition. The slow release composition comprises at least one slow release component and, in some embodiments, includes one (a single type of) slow release component. In further embodiments, the composition comprises at least two, different slow release components, each of which releases IGF-1 at a different rate or time after introduction into an individual by means of a stent or other surface coated with or otherwise bearing the composition comprising at least two, different slow release components . For example, a first slow release component releases the IGF-1 it contains within about 24 hours after introduction of the stent or other surface coated with or otherwise bearing the composition and a second, different slow release component releases the IGF-1 it contains over a longer period, such as 48 hours or 72 hours, or starting at a later time after introduction of the stent or other surface, such as starting at about 24 hours after introduction.

### BRIEF DESCRIPTION OF DRAWINGS

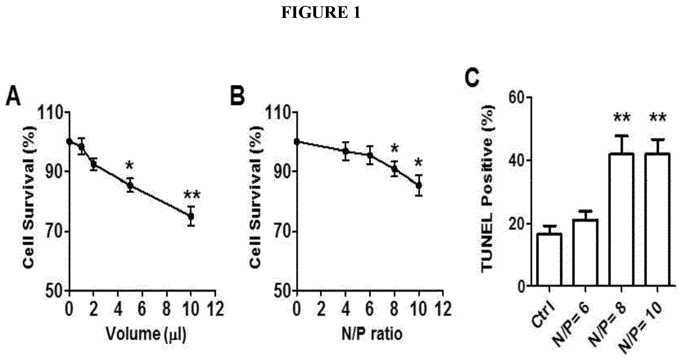

Figure 1A-C. Dose-toxicity study for PEI-modRNA nanoparticle. (A-B) HL-1 cells transfected with (A) varying volumes of jetPEI reagent and (B) varying N/P ratios of jetPEI/modGFP. Cell survival was monitored using cell counting with trypan blue exclusion staining. C) Quantification of TUNEL staining analysis for jetPEI-modGFP treated heart tissues. Heart slices were transfected with varying N/P ratios (6, 8, 10) of jetPEI-modGFP. Non-toxic 2 ml jetPEI volume and N/P ratio of 6 was used for subsequent in vivo modRNA-IGF1 experiments. *: p<0.05, **: p<0.001; Kruskal Wallis test, n= 3-4 per each group.

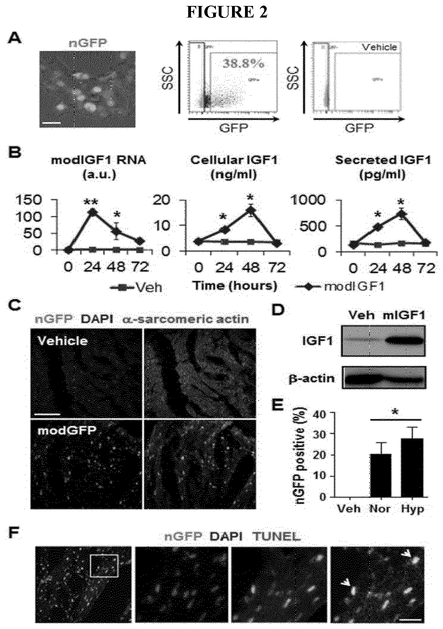

Figure 2A-F. Synthetic modRNA driven delivery and paracrine-like expression of IGF1 in CM. (A) HL-1 cardiomyocytes transfected with GFP modRNA (green: nuclear localising GFP, nGFP; scale bar: 20 µm) and nGFP expression quantified by FACS. B) Expression profiles of modRNA-IGF1 compared with vehicle (Veh) treated HL-1 cells. *: p<0.05, **: p<0.001, n=3 per treatment group. C) ModRNA-nGFP expression in mouse heart slices (green: nGFP, blue: DAPI, red: a-sarcomeric actin; scale bar: 50 µm). D) Immunoblot analysis of IGF1 protein in transfected heart slices (Veh: vehicle control, mIGF1: modRNA-IGF1). E) Percentage of nGFP positive cells in normoxic (Nor) and hypoxic (Hyp) conditions (Veh: vehicle control). *: p<0.05, n=6 per treatment group. F) Co-staining of nGFP and TUNEL positive signals (arrow heads) in merged last panel (green: nGFP, blue: DAPI, red: TUNEL positive; scale bar: 50 µm). 3 panels are magnified from inset.

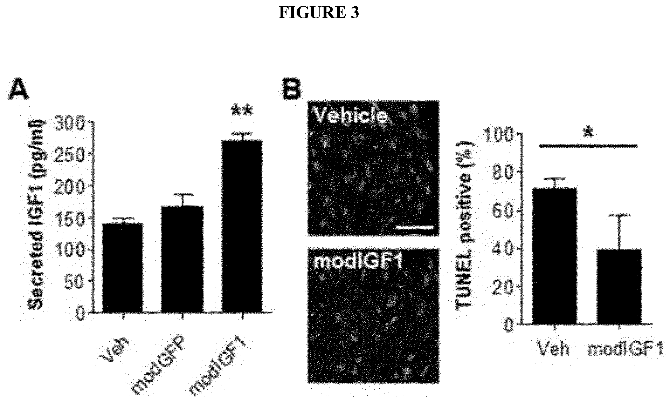

Figure 3A-B. ModRNA-IGF1 promoted in vitro CM survival. (A) Quantification of IGF1 secretion by ELISA. **: p<0.001, n=6 per treatment group. B) TUNEL staining analysis for modRNA-IGF1 treated heart tissue slices (red: TUNEL positive, blue: DAPI; scale bar: 20 µm). *: p<0.05, n=6 per treatment group.

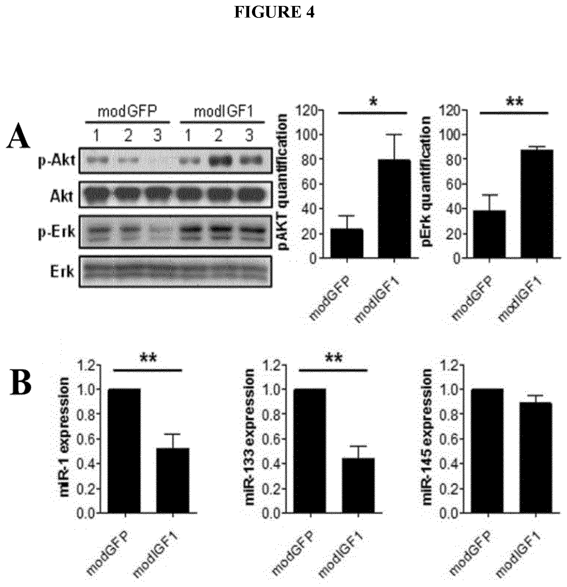

Figure 4A-B. ModRNA-IGF1 induced down-stream IGF1-associated signaling. (A) Immunoblot analysis and quantification of Akt and Erk phosphorylation in transfected heart slices. *: p<0.05, **: p<0.001, n=3 per treatment group. B) Expression profiles of miRNA-1, - 133, and -145 transcripts. **: p<0.001, n=3 per treatment group.

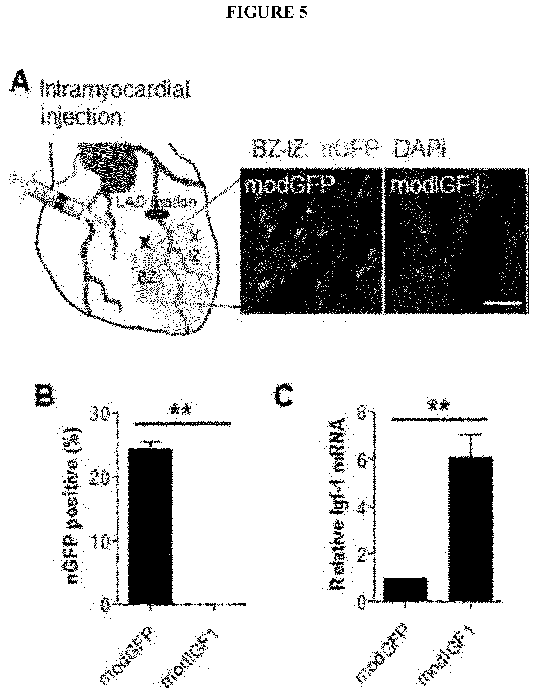

Figure 5A-C. Intramyocardial injection of modRNA-GFP and modRNA-IGF1 in infarct zone. (A) ModRNA-GFP expression is indicated in the infarct area post MI (BZ-IZ: border zone of infarct zone; green: nGFP, blue: DAPI; scale bar: 20 µm). x: sites of injection of modRNA. B) Percentage of nGFP positive cells in BZ-IZ. C) Expression of modRNA-IGF1 transcripts in BZ-IZ. **: p<0.001, n= 4 per treatment group.

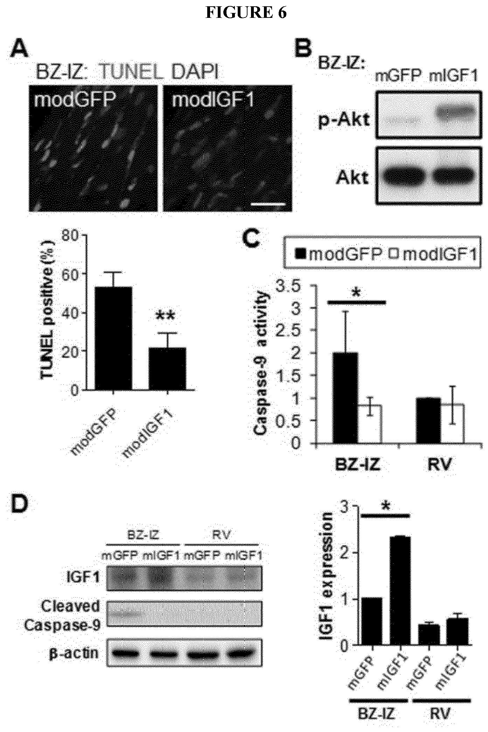

Figure 6A-D. In vivo cytoprotective effect of modRNA-IGF1 post MI. (A) TUNEL staining analysis for in vivo modRNA-IGF1 treated post MI heart tissues (red: TUNEL positive, blue: DAPI; scale bar: 20 µm). **: p<0.001, n= 4 per treatment group. B) Immunoblot analysis of Akt phosphorylation in heart tissues post MI. C) Caspase-9 activity analysis for heart tissues post MI. *: p<0.05, n= 4 per treatment group. D) Immunoblot analysis of IGF1 and active/cleaved caspase-9 expression in heart tissues post MI. BZ-IZ: border zone of infarct zone, RV: right ventricle, mGFP: modGFP, mIGF1: modIGF1; *: p<0.05, n= 3 per treatment group.

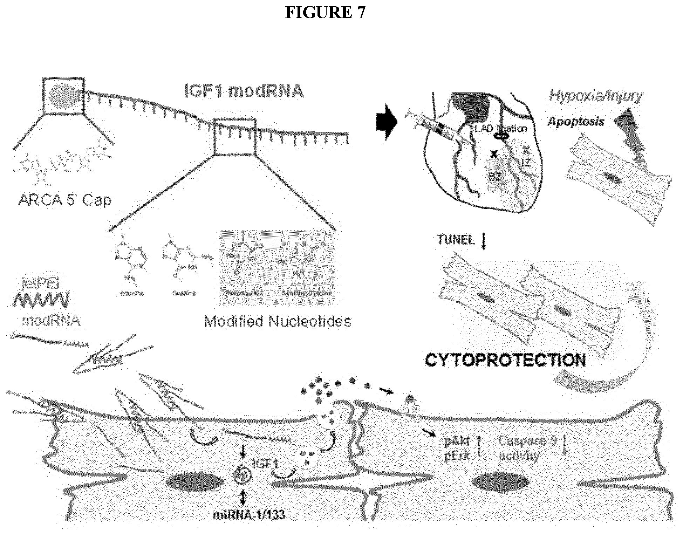

Figure 7 is a schematic of treating an MI using a modRNA of IGF-1.

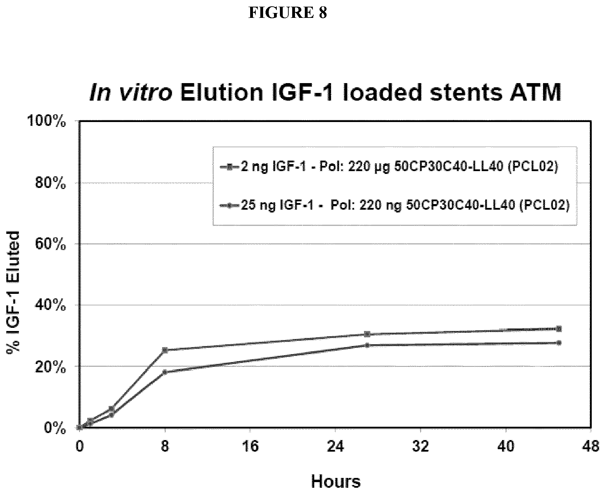

Figure 8 is a graph showing the % of IGF-1eluted over time for the stent coated with 2ng of IGF-1 and the stent coated with 25ng of IGF-1.

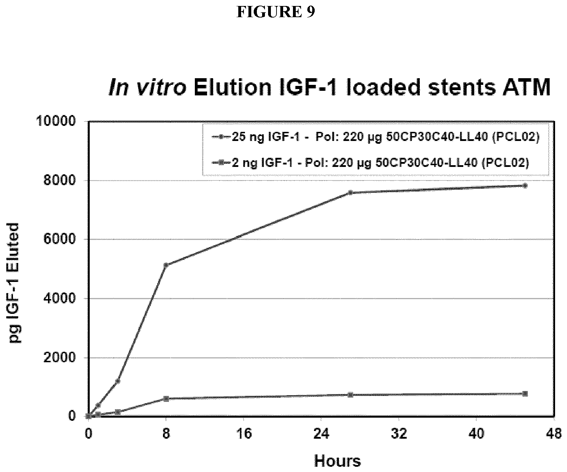

Figure 9 is a graph showing the total amount of IGF-1(pg) eluted over time for the stent coated with 2ng of IGF-1 and the stent coated with 25ng of IGF-1.

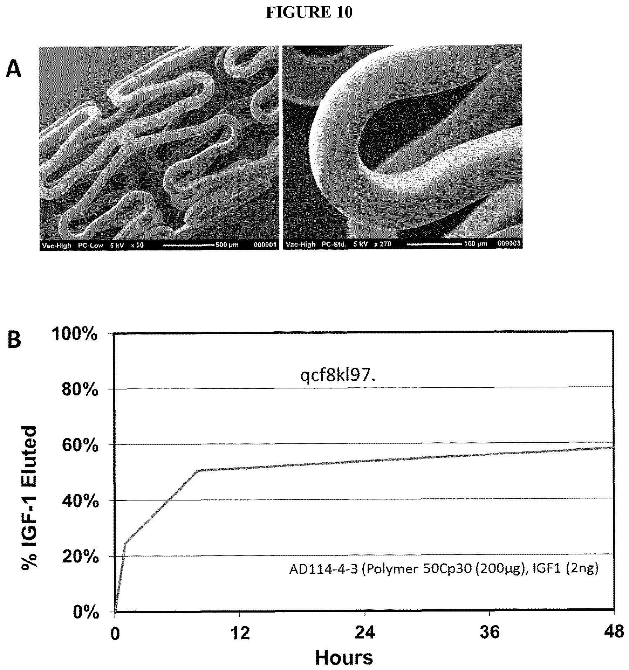

Figure 10A-B. (A) Shows SEM images of smooth surface of stent coated with (polymer 50Cp30 (200µg) and IGF1 2ng). (B) IGF-1 is slowly released from the polymer over 48 hours.

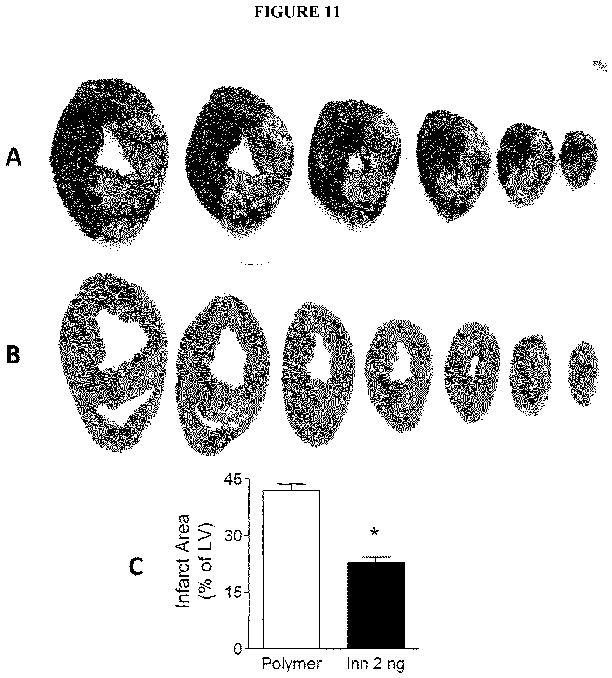

Figure 11A-C. Shows images of infarct size in heart sections of a pig MI model. Pigs treated with a stent coated with a slow-release polymer loaded with 2ng IGF-1 (B) showed a marked reduction of infarct size as compared to pigs treated with a stent coated with slow-release polymer (A). Infarct area quantified in (C).

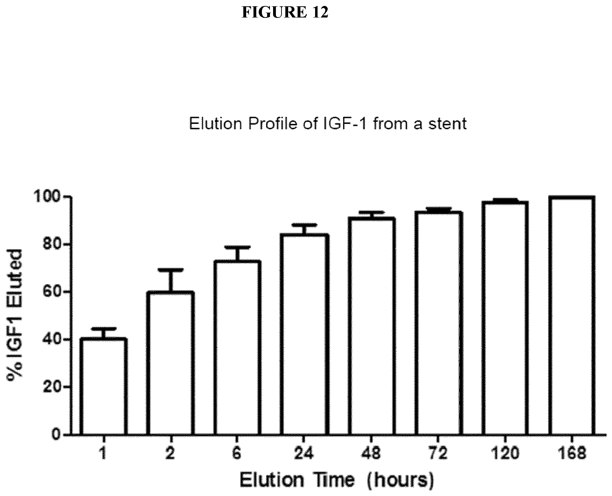

Figure 12 is a graph showing the elution profile of IGF-1 from TAZA stent. This relates to an atomic layer deposition (ALD) of aluminium oxide (Al₂O₃) linked with IGF1 peptide. This combination can be used to coat any stent, allowing the stent to elute IGF1 with a profile as shown in the figure.

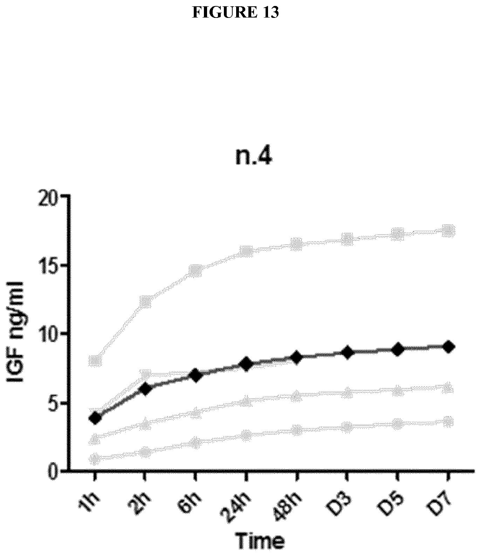

Figure 13 is a graph showing elution of IGF1 from a nanocoating of Aluminium Oxide (Al₂O₃) coated on a stent using atomic layer deposition (ALD). The IGF1 elutes in nanogram quantities over a time period (e.g., ≤72 hour time window, ≤48 hour time window, ≤24 hour time window) that is desirable to repair heart muscle after heart attack.

### DETAILED DESCRIPTION

Described herein is a slow release composition comprising IGF-1 and at least one (a, one or more) biocompatible slow release component useful for reducing cell death post myocardial infarction (in an individual who has had a myocardial infarction). Such a slow release composition is useful for cytoprotection of cells (cardiomyocytes) post myocardial infarction and for the production or manufacture of a therapeutic or a medicament for reducing cell death/cytoprotection of cells, such as cardiomyocytes post MI. Slow release compositions described herein can be administered by a variety of approaches, such as by injection (e.g., intracoronary injection, such as a single intracoronary injection) or through the use of (introduction into an individual of) a surface, such as a stent, coated with or otherwise having affixed thereto or bearing IGF-1 (e.g. in a slow-release composition comprising IGF-1 or coated with IGF-1 via ALD) in an amount or dose sufficient to cause a desired effect (cytoprotection of cells, such as cardiomyocytes, post myocardial infarction; reduction of cell death post myocardial infarction).

In some embodiments, the slow release composition comprises at least one component selected from the group consisting of: 50CP30L40-LL40 (PCL-02), Polyglycolide (PGA), Copolymers of glycolide, Glycolide/L-lactide copolymers (PGA/PLLA), Glycolide/trimethylene carbonate copolymers (PGS/TMC), Polylactides (PLA), Stereo-copolymers of PLA, Poly-L-lactide (PLLA), Poly-DL-lactide (PDLLA), L-lactide/DL-lactide copolymers, Copolyers of PLA, Lactide/tetramethylglycolide copolymers, Lactide/trimethylene carbonate copolymers, Lactide/a-valerolactone copolymers, Lactide/ε-caprolactone copolymers, Hyaluronic acid and its derivatives, Polydepsipeptides, PLA/polyethylene oxide copolymers, Unsymmetrical 3,6-substitute poly-1,4-dioxane-2,5-diones, Poly-phydroxybutyrate (PHBA), PHBA A/β-hydroxyvalerate copolymers (PHBA/HVA), Poly-p-dioxanone (PDS), Poly-α-valerolactone, Poly-e-caprolactone, Methylmethacrylate-N-vinyl pyrrolidine copolymers, Polyesteramides, Polyesters of oxalic acid, Polydihydropyranes, Polyalkyl-2-cyanoacrylates, Polyurethanes (PU), Polyvinylalcohol (PVA), Polypeptides, Poly-β-malic acid (PMLA), Poly-β-alcanoic acids and alginates.

In some embodiments, the slow release composition releases from about 1.0ng of IGF-1 to about 100ng of IGF-1 within 24 hours after introduction into an individual, such as by introduction of a stent bearing (e.g., coated with, having deposited thereon) the slow release composition or by injection of the slow release composition (e.g., by intracoronary injection).

In some embodiments, the slow release composition contains from about 1.0ng IGF-1 to about 100ng IGF-1. In further embodiments, the slow release composition releases from about 1.0ng IGF-1 to about 100ng IGF-1 into cardiac muscle within 48 hours after introduction into an individual (e.g., an individual who has had a MI) by introduction of a stent bearing (e.g., coated with, having deposited thereon) the slow release composition or by injection of the slow release composition (e.g., by intracoronary injection).

In further embodiments, the slow release composition releases from about 1.0ng-2.0ng, from about 1.0ng-5.0ng, from about 1.0ng-10ng, from about 1.0ng-20ng, from about 1.0ng-30ng, from about 1.0ng-40ng, from about 1.0ng-60ng, from about 1.0ng-80ng or from about 1.0ng-100ng IGF-1 within 48 hours after introduction into an individual (e.g., an individual who has had a MI) by introduction of a stent bearing (e.g., coated with, having deposited thereon) the slow release composition or by injection of the slow release composition (e.g., by intracoronary injection). In further embodiments, the slow release composition releases from 1.0ng - 15ng IGF-1 within 2 hours after intracoronary injection or introduction of a stent or other surface coated with or otherwise bearing the slow release composition.

The slow release composition can contain a single (one type of) slow release components or contain at least two (two or more), different slow release components. For example, the slow release composition can comprise one slow release component that releases from 1.0ng - 15ng IGF-1 within 2 hours after introduction into an individual (e.g., by introduction of a stent or other surface coated with or otherwise bearing the slow release composition or intracoronary injection) and a second, different slow release component that releases from 1.0ng - 100ng IGF-1 for up to 72 hours after introduction into an individual (e.g., by introduction of a stent or other surface coated with or otherwise bearing the slow release composition or intracoronary injection).

Also described herein are stents and other devices comprising (having affixed thereto, coated with or otherwise bearing) IGF-1, such as IGF-1 in a slow release composition that comprises IGF-1 and at least one (a, one or more) biocompatible slow release component useful for reducing cell death post myocardial infarction (in an individual who has had a myocardial infarction). Stents and other devices can have affixed thereto any slow release composition comprising IGF-1 and at least one (a, one or more) biocompatible slow release component described herein. Stents and other devices are coated with or have affixed thereto or bear IGF-1, such as IGF-1 in a slow-release composition comprising IGF-1 in an amount or dose sufficient to cause a desired effect (cytoprotection of cells, such as cardiomyocytes, post myocardial infarction; reduction of cell death post myocardial infarction).

In one embodiment, a drug eluting stent or other surface from which IGF-1 is released can be made by use of atomic layer deposition (ALD), which can be used to deposit IGF-1 or IGF-1 in a biocompatible polymer on a stent. ALD is used to coat Angstrom thick nano layers of biocompatible molecules that can be used for drug capture and controlled release of the captured drug, such as IGF-1. Briefly, ALD is a variant of chemical vapour deposition in which the chemical precursors required to form the desired layer are introduced separately, as part of a so-called ALD cycle. Growth then occurs via surface-limited chemistry such that usually only one or less than one monolayer of material is deposited per cycle. Accordingly, growth rates per cycle or GPC values are usually quoted in fractions of Angstroms. In one approach, the process involves the deposition of Al₂O₃ from the precursors, trimethyl aluminium and water. For example, one or more stents are placed into the ALD system, The system is evacuated to appropriate operating pressures (e.g., 0.3 mbar) and the cycle is initiated. During the first part of the cycle the trimethyl aluminium (TMA) is introduced. This reacts with the surface evolving methane and anchors a dimethyl aluminium species to the surface. This species then prevents further adsorption of TMA. A purge gas, such as N₂, is then introduced to flush away any excess gas phase TMA. At this point the second phase of the cycle in initiated by introducing water. The water reacts with the surface Al species, generating more methane and priming the surface for the adsorption of TMA during the next cycle.

The entire process of atomic layering is then repeated until the film reaches the desired thickness (e.g., for stents eluting IGF-1 an initial coating ranging from 1-10nm).This process yields a GPC of around 1 Angstrom, such that 10 full cycles are used in order to produce 1 nanometre of material. About 20 complete cycles are used to produce a layer of material 2 nanometers thick. In various embodiments, the layer of deposited material is from 0.5 nanometer to about 10 nanometers thick (e.g, 0.5 nanometer, 1 nanometer, 2 nanometers, 3 nanometers, 4 nanometers, 5 nanometers, 6 nanometers, 7 nanometers, 8 nanometers, 9 nanometers, 10 nanometers). After atomic layering of Al₂O₃ is complete, producing an Al₂O₃₋ coated stent or other surface, IGF-1 is adsorbed onto (on top of) the layer, from which it is released after insertion into an individual (e.g., into a coronary artery). Titanium oxide (TiO₂) can be coated/deposited onto the surface of a stent or other device using ALD, thus producing a stent or other device bearing titanium oxide which can be used to adsorb IGF-1 (a stent or other surface bearing titanium oxide-IGF-1). In some embodiments, ALD is used to produce aluminum oxide (Al₂O₃) coated nanoparticles, to which IGF-1 can be adsorbed, to produce nanoparticles coated with aluminum oxide and bearing IGF-1. Such nanoparticles are useful in the methods of cytoprotection of cells (cardiomyocytes) in an individual post myocardial infarction.

Since the ALD process is controlled by surface chemistry, it has the ability to coat both flat and highly textured, even porous surfaces, completely conformally, producing coatings which are free from pin-holes. (Johnson RW, Hultqvist A, Bent ST. A brief review of atomic layer deposition :from fundamentals to applications. Materials Today 2014:17(5):236-46) Titanium dioxide (TiO₂) and Aluminium Oxide (Al2O3) both of which have a long history as passivating nonreactive surfaces in vivo (hip replacements, orbital implants) can be used as chemistries to ALD coat any stent surface, including stainless steel or bioabsorbable polymer stents. (Hyde GK. et al, Atomic layer deposition and biocompatibility of titanium nitride nano-coatings on cellulose fiber substrates. Biomed Mater. 2009 Apr:4(2):025001; Finch DS et al., Biocompatibility of atomic layer-deposited alumina thin films. J Biomed Mater Res A. 2008 Oct;87(1):100-6) Within this coating conformation/design an anti-restenotic drug can be sandwiched between a TiO₂ layer that would stably react with the metal on the stent and an Al₂O₃ layer that would be the surface layer interacting with flowing blood when the stent is deployed in the vasculature in vivo. The drug can be loaded between these two layers and the kinetics of drug release would be in part determined by the release dynamics of drug determined by the rate of destruction by shear of the Al₂O₃ layer in vivo under arterial shear /flow conditions.

ALD coatings are orders of magnitude thinner than currently available polymer coatings used on 2nd generation DES (nm versus µm), which means that the profile of these coatings will likely be one of the lowest yet achieved in the DES industry. Their use can reduce or obviate the need for polymer coating of stents and, thus, reduce side effects that might occur with polymer retention/degradation on DES. The ALD process is sufficiently flexible that any stent scaffold or material can be coated with any number or combination of ALD nanolayers, which can be additionally functionalized with other therapeutic agents. For example, IGF-1 can be coated onto a stent or other surface using ALD. For example, the stent can be a single ALD Al₂O₃ coated conventional DES (DES-A-IGF1) or bioabsorable stent (BAS-A-IGF1) eluting an optimal dose of LD-IGF-1.

For example, aluminium oxide can be coated with IGF-1, such as IGF-1 in a slow release composition comprising IGF-1 and at least one biocompatible slow release component (e.g., any slow release composition described herein). In one embodiment, atomic layer deposition (ALD) of aluminium oxide is used. For example, atomic layer deposition of aluminium oxide linked with IGF-1 peptide is useful and this combination can be used to coat any stent, to produce a stent that elutes IGF-1 in an amount or a dose sufficient to cause a desired effect (cytoprotection of cells, such as cardiomyocytes, post myocardial infarction; reduction of cell death post myocardial infarction). As described herein, IGF-1 elutes from a stent, which can be any stent coated with aluminium oxide (e.g., by ALD). In a specific embodiment, the method of making the stent involves atomic layer deposition of aluminum oxide linked with IGF-1 peptide. This combination can be used to coat any stent, allowing the stent to elute IGF1 with a profile as shown in Figure 12.

Any stent, such as a drug eluting stent, from which IGF-1 can be released can be used. Examples include, but are not limited to stents such as those produced by Boston Scientific Corp (e.g., JACTAX stent, Taxus stent), Biosensors International (e.g., Champion stent, BioFreedom sent, BioMatrix stent), Cordis (e.g., Cypher), Medtronic (e.g., Endeavor), Abbott (e.g., Xience), Terumo Corp. (e.g., Nobori stent), Translumina GmbH (e.g., Yukon Choice stent), Sorin Group (e.g., Janus Flex stent), Minvasys (e.g., Amazonia Pax and Nile Pax). Alvimedica (e.g., Optima stent) and MIV Therapeutics (e.g.,VESTAsync). See also O'Brien, B. et al., Medical Stents: State of the Art and Future Directions in Annals of Biomedical Engineering. Published online 03 July 2015, incorporated herein by reference.

Also described are stents comprising nanocoating of aluminium oxide (Al₂O₃) applied to the stent using ALD. IGF1 elutes in nanogram quantities over a time period (e.g., ≤72 hour time window, ≤48 hour time window, ≤24 hour time window) that is desirable to repair heart muscle after heart attack.

ALD coated aluminium oxide is an elution platform for IGF1, such as a coating of nanoparticles or of stents. Described herein are stents coated with aluminium oxide (Al₂O₃) and IGF-1, from which IGF-1 elutes in an effective amount (an amount or a dose sufficient to cause a desired effect, such as cytoprotection of cells, such as cardiomyocytes, after myocardial infarction; reduction of cell death after myocardial infarction).

One embodiment of the present invention is a stent bearing aluminum oxide (Al₂O₃) and IGF-1, wherein the IGF-1 is adsorbed to the aluminum oxide. In a specific embodiment of the stent, the aluminum oxide is a coating from about 0.5 nm to about 10 nm in thickness. The aluminum oxide coating can be produced or placed on the stent or another surface by atomic layer deposition (ALD). A further embodiment is a stent bearing a coating of aluminum oxide (Al₂O₃) of thickness from about about 0.5 nm to about 10 nm. The stent, is some embodiments, further comprises IGF-1 adsorbed to the aluminum oxide (Al₂O₃).

### EXAMPLE

### IGF-1 Coating of Stents

Three batches of coated stents are designated as follows:
1. Polymer only coated stents: 200µg 50CP30C40-LL40 (PCL-02)
2. 2 ng IGF-1 in 200µg 50CP30C40-LL40 (PCL-02)
3. 20 ng IGF-1 in 200µg 50CP30C40-LL40 (PCL-02)

### Treating Myocardial Infarction (MI) with IGF-1 Coated Stents

Results described herein show that slow release of IGF1 from a polymer starting at a 2ng dose of IGF1 is effective in reducing infarct size by 50% post MI (Figures 10 and 11).

## Claims

1. A slow release composition comprising IGF-1 and at least one biocompatible slow release component, wherein the composition is configured to release from about 1.0ng of IGF-1 to about 100ng of IGF-1 within 24 hours after injection.

2. The slow release composition of claim 1, wherein the composition comprises at least one component selected from the group consisting of: 50CP30L40-LL40 (PCL-02), Polyglycolide (PGA), Copolymers of glycolide, Glycolide/L-lactide copolymers (PGA/PLLA), Glycolide/trimethylene carbonate copolymers (PGS/TMC), Polylactides (PLA), Stereo-copolymers of PLA, Poly-L-lactide (PLLA), Poly-DL-lactide (PDLLA), L-lactide/DL-lactide copolymers, Copolyers of PLA, Lactide/tetramethylglycolide copolymers, Lactide/trimethylene carbonate copolymers, Lactide/a-valerolactone copolymers, Lactide/ε-caprolactone copolymers, Hyaluronic acid and its derivatives, Polydepsipeptides, PLA/polyethylene oxide copolymers, Unsymmetrical 3,6-substitute poly-1,4-dioxane-2,5-diones, Poly-phydroxybutyrate (PHBA), PHBA A/β-hydroxyvalerate copolymers (PHBA/HVA), Poly-p-dioxanone (PDS), Poly-α-valerolactone, Poly-e-caprolactone, Methylmethacrylate-N-vinyl pyrrolidine copolymers, Polyesteramides, Polyesters of oxalic acid, Polydihydropyranes, Polyalkyl-2-cyanoacrylates, Polyurethanes (PU), Polyvinylalcohol (PVA), Polypeptides, Poly-β-malic acid (PMLA), Poly-β-alcanoic acids and alginates.

3. The slow release composition of Claim 1 or 2-4, wherein the IGF-1 content is from about 1.0ng IGF-1 to about 100ng IGF-1.

4. The slow release composition of Claim 1, 2 or 3, wherein the composition is configured to release from about 1.0ng IGF-1 to about 100ng IGF-1 into cardiac muscle within 48 hours after intracoronary injection.

5. The slow release composition of any of claims 1 to 4, wherein the composition is configured to release from about 1.0ng-2.0ng, from about 1.0ng-5.0ng, from about 1.0ng-lOng, from about 1.0ng-20ng, from about 1.0ng-30ng, from about 1.0ng-40ng, from about 1.0ng-60ng, from about 1.0ng-80ng or from about 1.0ng-100ng IGF-1 within 48 hours after intracoronary injection.

6. The slow release composition of any of claims 1 to 5, wherein the composition is con figured to release from 1.0ng - 15ng IGF-1 within 2 hours after intracoronary injection.

7. The slow release composition of any of claims1 to 6, wherein the composition comprises at least two, different slow release components.

8. The slow release composition of claim 7, wherein one slow release component is configured to release from 1.0ng - 15ng IGF-1 within 2 hours after intracoronary injection and a second, different slow release component is configured to release from 1.0ng - 100ng IGF-1 for up to 72 hours after intracoronary injection.

9. The slow release composition of any of Claims 1 to 8, for use in a method of reducing cell death in heart muscle/tissue resulting from myocardial infarction, comprising intracoronary injection of the slow release composition in an individual (in whom a myocardial infarct has occurred) within 72 hours after occurrence of the myocardial infarct, wherein the therapeutically effective amount is an amount that reduces the extent to which cell death occurs in heart muscle/tissue.

10. The slow release composition of any of Claims 1 to 8, for use in a method of reducing cell death in heart muscle/tissue resulting from myocardial infarction, comprising administering to an individual a single intracoronary injection of the slow-release composition , within 72 hours after myocardial infarction and in a location that results in delivery of IGF-1 to the area of risk in an amount sufficient to reduce the extent to which cell death occurs in heart muscle/tissue.

11. The slow release composition of any of Claims 1 to 8, for use of any of Claims 9 or 10, wherein the slow release composition is injected within 5 minutes, within 10 minutes, within 30 minutes, within 1 hour, within 2 hours, within 5 hours, within 8 hours, within 12 hours, within 18 hours, within 24 hours, within 36 hours, within 48 hours, within 60 hours or within 72 hours after occurrence of myocardial infarct.

12. The slow release composition of any of Claims 1 to 8, for use of any of Claims 9 or 10, wherein IGF-1 is administered within 24 to 48 hours after occurrence of myocardial infarct.

13. The slow release composition of any one of claims 1-8 for use as a medicament.
